# EUROPEAN PATENT SPECIFICATION

(11) **EP 0 906 335 B1**
(45) Date of publication and mention of the grant of the patent: **13.02.2008**
(21) Application number: 97921544.9
(22) Date of filing: 23.05.1997
(51) Int. Cl.: C07K 7/06, A61K 39/245

(54) **EBV TYPE B CTL EPITOPES**
EPSTEIN-BARR VIRUS TYPE B CTL EPITOPEN
EPITOPES DE LYMPHOCYTES T DU VIRUS D'EPSTEIN-BARR TYPE B

(30) Priority: 24.05.1996 AU PO007396
(43) Date of publication of application: 07.04.1999
(73) Proprietor: THE COUNCIL OF THE QUEENSLAND INSTITUTE OF MEDICAL RESEARCH, Herston, QLD 4029 (AU); COMMONWEALTH SCIENTIFIC AND INDUSTRIAL RESEARCH ORGANISATION, Campbell, ACT 2612 (AU); The University of Melbourne, Parkville, VIC 3052 (AU); THE WALTER AND ELIZA HALL INSTITUTE OF MEDICAL RESEARCH, Parkville, VIC 3052 (AU); CSL LIMITED, Parkville, VIC 3052 (AU)
(72) Inventor: KHANNA, Rajiv, Herston, QLD 4006 (AU); KERR, Beverley, Mavis, Gumdale, QLD 4154 (AU); MISKO, Ihor, Spephan, St. Lucia, QLD 4067 (AU); MOSS, Denis, James, Arana Hills, QLD 4054 (AU); BURROWS, Scott, Renton, Bald Hills, QLD 4036 (AU)
(74) Representative: Bassil, Nicholas Charles
(86) International application number: PCT/AU1997/000328
(87) International publication number: WO 1997/045444

(56) References cited:
- WO-A-91/08224
- WO-A-95/24925
- APOLLONI A ET AL: "SEQUENCE VARIATION OF CYTOTOXIC T CELL EPITOPES IN DIFFERENT ISOLATES OF EPSTEIN-BARR VIRUS" EUROPEAN JOURNAL OF IMMUNOLOGY, WEINHEIM, DE, vol. 22, 1992, pages 183-189, XP001007889 ISSN: 0014-2980
- KERR B M ET AL: "IDENTIFICATION OF TYPE B-SPECIFIC AND CROSS-REACTIVE CYTOTOXIC T-LYMPHOCYTE RESPONSES TO EPSTEIN-BARR VIRUS" JOURNAL OF VIROLOGY, THE AMERICAN SOCIETY FOR MICROBIOLOGY, US, vol. 70, no. 12, December 1996 (1996-12), pages 8858-8864, XP001007896 ISSN: 0022-538X
- JOURNAL OF VIROLOGY, December 1996, Volume 70, Number 12, KERR B.M. et al., "Identification of Type B-Specific and Cross-Reactive Cytotoxic T-Lymphocyte Responses to Epstein-Barr Virus", pages 8858-8864. XP001007896
- JOURNAL OF VIROLOGY, August 1990, Volume 64, Number 8, BURROWS S.R. et al., "An Epstein-Barr Virus-Specific Cytotoxic T-Cell Epitope Present on A- and B-Type Transformants", pages 3974-3976. XP001007893

## Description

### FIELD OF THE INVENTION

The present invention relates to cytotoxic T-cell (CTL) epitopes within Epstein-Barr virus. The present invention also relates to subunit vaccines including CTL epitopes.

### BACKGROUND OF THE INVENTION

Epstein-Barr virus (EBV) is a gamma herpesvirus which establishes a latent lifelong infection in the host following acute infection (14,15). While primary infection generally occurs in childhood without significant morbidity, adolescents and young adults may present with the symptoms of acute infectious mononucleosis (IM). The main feature of IM is a self-limiting lymphoproliferation involving both T and B cells accompanied by clinical symptoms such as fever and lymphadenopathy (52.53). Occasionally, the clinical symptoms persist and recur for extended periods after the initial infection. Episodic IM such as this has been described as chronic active EBV infection or, in some cases, severe chronic active EBV infection (35). EBV DNA has been detected in both serum and peripheral blood lymphocytes (PBL) during acute IM with the levels of detectable DNA gradually decreasing as the illness abates (21.22.58).

Evidence for latent EBV infection includes the observation that spontaneous lymphoblastoid cell lines (LCLs), expressing latent proteins, can be regularly established from healthy immune individuals after explantation of either lymph node tissue (34) or fractionated B lymphocytes (59). Although latent EBV infection is usually asymptomatic, sequential studies have established that recrudescence of viral replication in the oral cavity may result in release of infectious virus (59). The exact site of persistence of the virus is uncertain, but the available evidence suggests that small lymphocytes in the circulation harbour the virus in a nonproductive episomal state (24). Accordingly, in asymptomatic donors. EBV DNA is detectable by sensitive PCR analysis in PBL expressing the B-cell marker CD19 (29,55).

EBV is also involved in post transplant lymphoproliferative disease, which involves a polyclonal expansion of EBV infected B-cells which is a life threatening lymphoma especially in transplantation patients. EBV is also involved in nasopharyngeal carcinoma and Hodgkinson's disease.

Two types of EBV (types A and B or types 1 and 2) are distinguishable primarily on the basis of variation in the DNA and protein sequences of the latent EBV-associated nuclear antigens (referred to here as EBNAs-2A, -3A, -4A and -6A from the type A virus and EBNAs-2B, -3B, -4B and -6B from the type B virus) (9,46,48). Sequencing of the prototypical isolates of type A and type B EBV (B95-8 and Ag-876 respectively) in these regions revealed 53% amino acid homology between EBNA-2A and EBNA-2B (9) and 72-84% homology between EBNAs-3A and -3B, EBNAs-4A and - 4B, and EBNAs-6A and -6B (46). Strain variation due to other DNA alterations or deletions as well as these A/B type differences have been defined at the protein (12) and the DNA level (16,25,26) and recombination between multiple infecting strains was found to occur frequently in oral hairy leukoplakia lesions (56). These variations offer an alternative means of categorising EBV isolates but the primary distinction of type A and type B is still useful. Type A EBV is more readily isolated from healthy donors; type B EBV infections or dual infections with both type A and type B have proven easier to detect in immunosuppressed or HIV infected individuals (5,47,51). A higher incidence of type B infection in some studies led to the suggestion that type B or dual infections are, in fact, relatively common and that resident type B virus levels increase during immunosuppression (3,20,51).

It appears that latent EBV infection is primarily controlled by HLA class I-restricted memory cytotoxic T cell (CTL) responses (reviewed in (18)). These CTL responses can be reactivated in vitro by stimulating lymphocytes from seropositive individuals with autologous lymphoblastoid cell lines (LCLs) which express and present MHC class I and class II restricted epitopes at the cell surface. Several of these epitopes have been identified using target cells infected by recombinant vaccinia constructs (17,19,32,33). Epitopes specific for type A EBV as well as cross-reactive epitopes encoded by both types A and B EBV have been defined (18) but no epitopes specific for type B EBV have been reported thus far.

International Patent Publication No. WO91/08224 discloses several synthetic peptides derived from the predicted amino acid sequence of the EBV genome, and their use in an antibody-based ELISA test for the diagnosis of infectious mononucleosis (1M) and related diseases. However, as WO91/08224 was interested only in antibody responses, they did not mention or suggest that any epitope was a CTL epitope. Nor did WO91/08224 mention or suggest CTL epitopes specific for type B EBV.

Apolloni et al., Eur J. immunol. 22, 183-189, 1992 examined the possibility that cytotoxic T lymphocytes may exert immune pressure on EBV, thereby resulting in variation within regions encoding the CTL, in relation to the variation between Type A EBV and Type B EBV in gene regions coding for the CTL epitopes TETAQAWNAGFLRGRAYGIDLLRTE ("TETA") of EBNA3 and EENLLDFVRFFMGVMSSCNNP ("EENL") of EBNA6. Sequencing of DNA regions encoding these CTL epitopes in different EBV isolates demonstrated no variations in the EENL epitope between Type A and Type B EBV strains.. In contrast, there were variations in the TETA epitope between Type A and Type B EBV, in which the variants from Type B EBV strains possessed no activity as a CTL epitope. The authors concluded that substantial divergence and differences in biological characteristics make it unlikely that the variations arose as a consequence of immune pressure and suggested an evolutionary divergence that gave rise to Type A and Type B EBV.

International Patent Publication No. WO95/24925 (PCT/AU95/00140) discloses several CTL epitopes from Epstein-Barr virus latent antigens. The CTL epitopes have the following amino acid sequences: QAKWRLQTL, RYSIFFDY, HLAAQGMAY, YPLHEQHGM, YPLHKQHGM, YRLHEQHGM, YPLHEQRGM, SVRDRLARL, AVLLHEESM, TVLLHEESM, TALLHEESM, VSFIEFVGW, FRKAQIQGL, PYLFWLAAI, TVFYNIPPMPL, PGDQLPGFSDGRACPV, and VEITPYKPTW.

In the present study, the response of a donor exposed to both type A and type B EBV was investigated and an epitope specific for type B EBV as well as a new cross-reactive epitope were identified.

### SUMMARY OF THE INVENTION

In a first aspect the present invention consists in a cytotoxic Epstein-Barr virus T-cell epitope, the epitope being selected from the group consisting of QVKWRMTTL and QNGALAINTF.

In a second aspect the present invention consists in an EBV type B-specific subunit vaccine, the vaccine including at least one T-cell epitope selected from the group consisting of QVKWRMTTL and QNGALAINTF.

In a preferred embodiment of this aspect of the present invention the EBV type B-specific subunit vaccine includes the T-cell epitopes QVKWRMTTL and QNGALAINTF.

In a further preferred form of the present invention the vaccine comprises a water-in-oil formulation. It is further preferred that the vaccine includes at least one antigen to which the individual will mount an anamnestic response in addition to the at least one cytotoxic T-cell epitope.

The at least one antigen is preferably selected from the group consisting of tetanus toxoid, diphtheria toxoid, Bordetella pertussis antigens, poliovirus antigens, purified protein derivative (PPD), gp350 protein (Thorley-Lawson, D.A. and Poodry, C.A. (1982). Identification and isolation of the main component (gp350-gp220) of Epstein-Barr virus responsible for generating neutralizing antibodies in vivo, J. Virol. 43, 730-736), helper epitopes and combinations thereof and is preferably tetanus toxoid.

It is preferred that the water-in-oil formulation is Montanide ISA 720. Additional information regarding this formulation can be found in WO 95/24926, the disclosure of which is incorporated herein by cross reference.

The subunit vaccine may also be formulated using ISCOMs. Further information regarding ISCOMs can be found in Australian Patent Nos, 558258, 590904, 632067, 589915, the disclosures of which are included herein by cross reference.

In a third aspect the present invention consists in a nucleic acid vaccine, the vaccine including a nucleic acid sequence encoding at least one of the cytotoxic T-cell epitopes of the first aspect of the present invention and preferably, both epitopes.

Further information regarding nucleic acid vaccines can be found in WO 96/03144 the disclosure of which is incorporated herein by cross reference. As will be appreciated by those skilled in the field the DNA can be delivered using a suitable viral or bacterial vector.

The vaccines of the present invention may be used prophylactically or therapeutically.

### DETAILED DESCRIPTION OF THE INVENTION

The CTL epitopes may be synthesised using techniques well known to those skilled in this field. For example, the CTL epitopes may be synthesised using solution synthesis or solid phase synthesis as described, for example, in Chapter 9 entitled "Peptide Synthesis" by Atherton and Sheppard which is included in a publication entitled "Synthetic Vaccines" edited by Nicholson and published by Blackwell Scientific Publications. Preferably a solid phase support is utilised which may be polystyrene gel beads wherein the polystyrene may be cross-linked with a small proportion of divinylbenzene (e.g. 1%) which is further swollen by lipophilic solvents such as dichloromethane or more polar solvents such as dimethylformamide (DMF). The polystyrene may be functionalised with chloromethyl or anionomethyl groups. Alternatively, cross-linked and functionalised polydimethyl-acrylamide gel is used which may be highly solvated and swollen by DMF and other dipolar aprolic solvents. Other supports can be utilised based on polyethylene glycol which is usually grafted or otherwise attached to the surface of inert polystyrene beads. In a preferred form, use may be made of commercial solid supports or resins which are selected from PAL-PEG, PAK-PEG, KA. KR or TGR.

In solid state synthesis, use is made of reversible blocking groups which have the dual function of masking unwanted reactivity in the a-amino, carboxy or side chain functional groups and of destroying the dipolar character of amino acids and peptides which render them inactive. Such functional groups can be selected from t-butyl esters of the structure RCO-OCMe₃-CO-NHR which are known as t-butoxy carboxyl or ROC derivatives. Use may also be made of the corresponding benzyl esters having the structure RCO-OCH₂-C₆H₅ and urethanes having the structure C₆H₅CH₂O CO-NHR which are known as the benzyloxycarbonyl or Z-derivatives. Use may also be made of derivatives of fluorenyl methanol and especially the fluorenyl-methoxy carbonyl or Fmoc group. Each of these types of protecting group is capable of independent cleavage in the presence of one other so that frequent use is made, for example, of BOC-benzyl and Fmoc-tertiary butyl protection strategies.

Reference also should be made to a condensing agent to link the amino and carboxy groups of protected amino acids or peptides. This may be done by activating the carboxy group so that it reacts spontaneously with a free primary or secondary amine. Activated esters such as those derived from p-nitrophenol and pentafluorophenyl may be used for this purpose. Their reactivity may be increased by addition of catalysts such as 1-hydroxybenzotriazole. Esters of triazine DHBT (as discussed on page 215-216 of the abovementioned Nicholson reference) also may be used. Other acylating species are formed in situ by treatment of the carboxylic acid (i.e. the Na-protected amino acid or peptide) with a condensing reagent and are reacted immediately with the amino component (the carboxy or C-protected amino acid or peptide). Dicyclohexylcarbodiimide, the BOP reagent (referred to on page 216 of the Nicholson reference), O'Benzotriazole-N, N. N'N'-tetra methyl-uronium hexaflurophosphate (HBTU) and its analogous tetrafluroborate are frequently used condensing agents.

The attachment of the first amino acid to the solid phase support may be carried out using BOC-amino acids in any suitable manner. In one method BOC amino acids are attached to chloromethyl resin by warming the triethyl ammonium salts with the resin. Fmoc-amino acids may be coupled to the p-alkoxybenzyl alcohol resin in similar manner. Alternatively, use may be made of various linkage agents or "handles" to join the first amino acid to the resin. In this regard, p-hydroxymethyl phenylactic acid linked to aminomethyl polystyrene may be used for this purpose.

Details of the CTL epitopes of the present invention are set in Tables 3 and 4.

As will be readily appreciated by those skilled in the art the cytotoxic T-cell epitopes and vaccines of the present invention can be used to protect against EBV. Further given the possible greater involvement of type B EBV infection in immunocompromised individuals the present invention may have particular application in protection of individuals having decreased immune function, eg transplant patients.

In order that the nature of the present invention may be more clearly understood preferred forms thereof will now be described with reference to the following examples and Figures.

### FIGURE LEGENDS

**Fig 1****.** Immunoblot of recombinant vaccinia-EBNA viruses: Lane 1: vacc-TK-, Lane 2: vacc-EBNA-3B; Lane 3: vacc-EBNA-6B. M. Wt. markers are on the left.
**Fig. 2**. Confirmation of specificity of CTL cultures B5 and A3. EW-Wil (type A autologous LCL) was infected with vacc- EBNA-6A, vacc-EBNA-6B or vacc-TK and used as targets in a CTL assay; EW-Wil and EW-Ag-876 (type B autologous LCL) were used as control targets without vaccinia infection.
**Fig. 3a****, b**: Identification of minimal epitope for culture B5. Targets: EW-Wil LCL +peptide

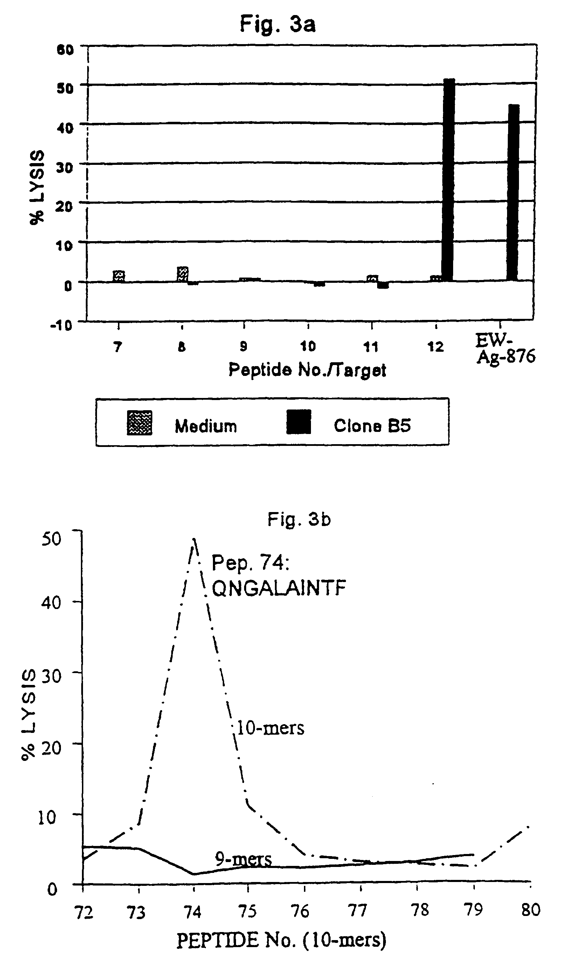

**Fig. 3a****:** Targets + peptides 7-12 of EBNA 6B 20-mer overlap
**Fig. 3b****:** Targets +9-mer and 10-mer overlapping peptides of the 20-mer peptide No. 12

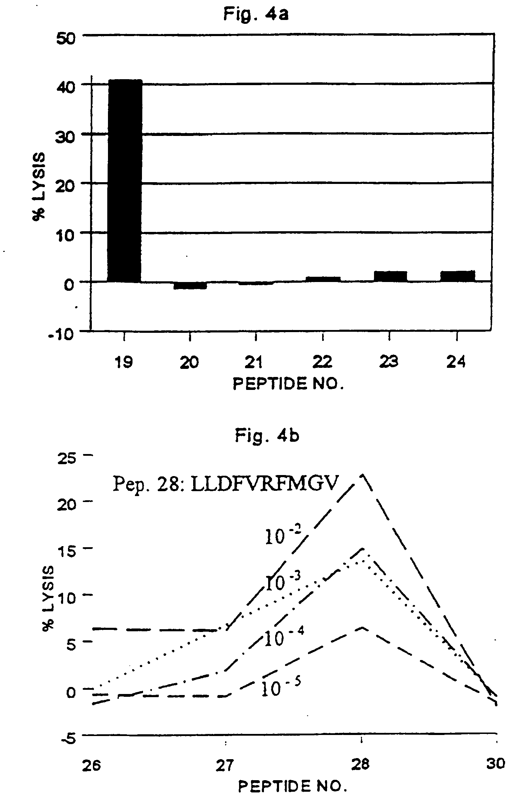

**Fig. 4a****, b**: Identification of minimal epitope for culture A3. Targets: EW-PHA blasts + peptide
**Fig. 4a**: Targets + peptides 19-24 of EBNA-6B 20-mer overlap
**Fig. 4b**: Targets + dilutions of four of the 10-mer overlapping peptides of the 20-mer peptide No. 19

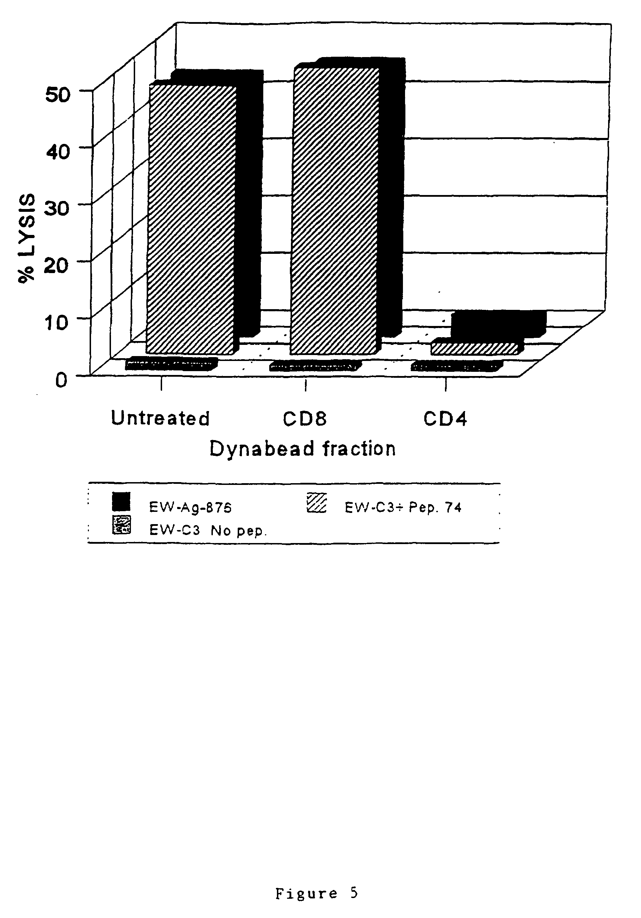

**Fig. 5**: Depletion experiment. Targets: EW-C3 (negative T cell culture); Effectors: EW type B epitope specific cultures pooled from LDA cultures. EW = CTL treated with Dynabeads only: EW-CD-8: CTL depleted of CD4 T cells: EW-CD4: CTL depleted of CD8 T cells

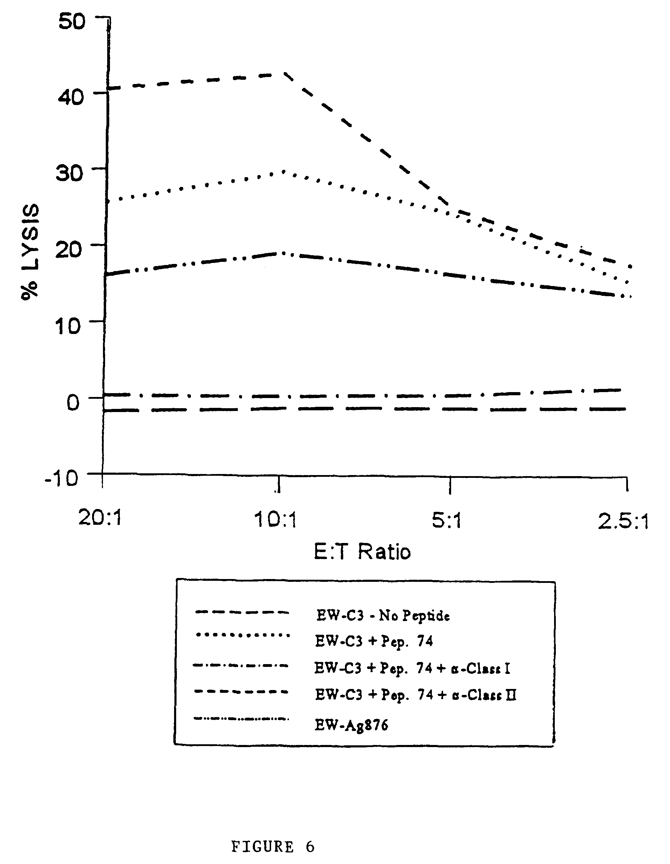

**Fig. 6****.** Inhibition of type B epitope-positive CTL (culture B-15) by a-Class I but not by a-Class II monoclonal antisera. Peptide 74 = type B reactive peptide; target cells = EW-C3, a noncytolytic T cell culture.

### MATERIALS AND METHODS

### Lymphocyte donor

A 17 year old individual (EW) who initially presented with clinical and pathological (EBV IgM positive) symptoms of acute IM was used in this study. She was referred to this laboratory for investigation of her EBV status 7 months after the initial diagnosis as her clinical symptoms persisted. Four bleeds were obtained from this donor over a period of 12 months during which time the symptoms fluctuated but did not completely resolve. The third and fourth bleeds were used for CTL analysis.

### Virus Typing and Detection

DNA was extracted from PBL by a small scale adaptation of a published extraction procedure (27). EBNA 2A and 2B primers as previously described (20) were used to distinguish between type A and type B EBV by PCR. Briefly, standard PCR reactions were carried out in a 25 µl volume using 1 µg genomic DNA. The template was denatured for 2 min at 94°C before subjecting to 35 PCR cycles (15 s at 94°C. 30 s at 56°C and 15 s at 72°C). PCR amplified products were identified on southern blots using digoxigenin-dUTP (DIG) labelled DNA probes (Boehringer-Mannheim Australia ) generated by PCR using the same primers as above and either the purified M-ABA Bam HI insert from pM-Bam H2 (type A) (36) or the purified Jijoye Bam HI/Pst I insert from pJ-HKA7 (type B) (1) as templates. For the preparation of the probes, DIG-dUTP was present in the PCR reaction with 25 ng of each insert as template.

### Vaccinia constructs

Recombinant vaccinia constructs (designated vacc-EBNA-1, vacc-EBNA-2 etc) encoding EBNAs -1, -2B, -4A and -6A, the latent membrane proteins LMP1 and LMP2, the lytic antigens BHRF1 and BZLF1, and the control vacc-TK⁻ have already been described (19.23.33). Vacc- EBNA-3B and vacc- EBNA-6B (i.e. vaccinia constructs encoding the type B sequences of EBNA 3 and EBNA 6 respectively) were constructed from an EBV genomic library derived from purified viral genomes of the prototype type B strain Ag-876 (50). The viral DNA was digested with HindIII and ligated into the λ-ZAP cloning system (Integrated Sciences).

An 11.07 kbp HindIIIE fragment encoding the EBNA 3 family open reading frames (ORFs) was subcloned into the vector pUC19. A 2.6 kbp BamHI-CEIII (Klenow-treated) fragment within the region containing the BERF1 ORF of EBNA-3 (46) was isolated and ligated into a BamHI-HindII digest of pBCB07. This vaccinia vector drives recombinant gene expression by the 7.5K vaccinia virus promoter (2). The EBNA-3B sequence encoded in the transfer plasmid was inserted into vaccinia virus by transfection and homologous recombination into the TK gene of vaccinia as described (2). A 4 kbp Eco47III-HindIII fragment within the EBV HindIIIE region containing the BERF2b ORF of EBNA-6B was isolated and ligated into a HindII-HindIII digest of pBCB07. This was subsequently processed as for EBNA-3B.

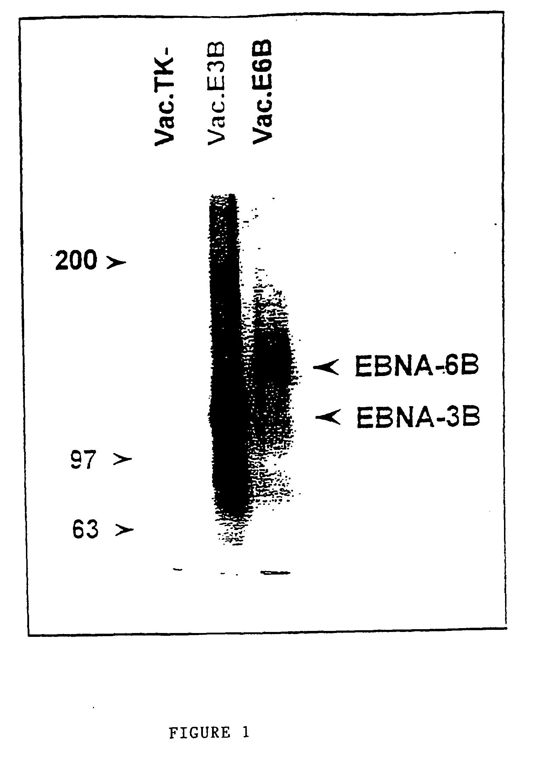

Integrity of the vacc-EBNA-3B and -6B constructs was confirmed by immunoblot after infection of mouse CV-1 cells. 2x10⁵ CV-1 cells infected with control vaccinia (vacc-TK⁻). vacc-3B or vacc-6B were lysed in protein sample buffer and separated on a 7% SDS-polyacrylamide gel in a Mini-protean II Cell System (Bio-Rad). After transferring to nitro-cellulose the blot was incubated with a multivalent human serum (MCr) which contained EBNA-specific antibodies. The reactions were visualised with the ECL detection system (Amersham). Fig. 1 illustrates the production of a 110 kDa protein (EBNA-3B) or a 130 kDa protein (EBNA-6B) corresponding to the expected products of EBNA-3B and EBNA-6B without the short first ORFs. Sequence analysis also confirmed the integrity of the constructs.

### Establishment of cell cultures

(a) B Cell Cultures: LCLs were established from EW by exogenous transformation of PBL by QIMR-WIL virus (type A; (38)) and Ag-876 (type B). These LCLs were designated EW-WIL and EW AG-876. Spontaneous LCLs (i.e. autologous LCLs infected with the donor's endogenous virus strain and designated EW-spon) were established in the presence of cyclosporin (41). LCLs from other donors were transformed by QIMR-WIL. B95-8 or IARC-BL74 virus (30) (type A isolates) or by Ag-876 or QIMR-GOR virus (37) (type B isolates). Other lines used in this study include Burkitt lymphoma cell lines (Chep and Mutu) and the spontaneous line QIMR-WW2-LCL (13.43.44). B cell lines were maintained in RPMI 1640 with 10-20%FCS.
(b) T Cell Cultures: Unfractionated PBL were separated on Ficoll-Paque (Pharmacia. Uppsala, Sweden) and stimulated by incubating with γ-irradiated (8000 rads) autologous LCLs (EW-Wil or EW-Ag-876: 2 x 10⁶ cells per well in 2 ml growth medium without rIL-2. responder:stimulator ratio 50:1) (28). After 3 days, the cultures were cloned in agarose (Seaplaque, FMC Corp. Rockland ME) and the colonies harvested 3-5 days later. Once established. the T cells were maintained in T cell growth medium (RPMI-1640 supplemented with 15% FCS. 30% MLA-144 supernatant (TIB-201: American type Culture Collection. Rockville. MD and rIL-2. 20 units/ml) (45.57). Since the clonal nature of the T cells was not established, they are referred to in the text as cultures rather than clones. The cultures were routinely maintained in either 24-well plates or tissue culture flasks and restimulated twice weekly with γ-irradiated (8000 rads) autologous LCLs.

Bulk T cell cultures were initially stimulated from the fourth bleed as for cloning but subcultured and maintained after 3-4 days as for T cell colonies. Phytohaemagglutinin (PHA) blasts were generated by stimulation of PBL with PHA (CSL, Melbourne, Australia) and subculturing after 4 days in T cell growth medium. Cultures were regularly screened for mycoplasma contamination.

### FACS analysis

Three-colour cytofluorographic analysis of T cell cultures was performed by means of direct immunofluorescence using labelled monoclonal antibodies specific for CD3 (tricolour- conjugated). CD4 (fluorescein -conjugated), and CD8 (phycoerythrin-conjugated) and (Becton Dickinson. California). The labelled cells were analysed on a FACScan (Becton Dickinson, lysis II software).

### Cytotoxicity assays

T cells were assayed in modified 5-hour ⁵¹Cr-release assays (30). Briefly, target cells were labelled for 1 hour with 0.5-1.0µC ⁵¹Cr. washed and resuspended at 10⁵ cells/ml. 50µl of target cells were incubated with an equal volume of effectors (Effector:target or E:T ratio generally 10:1) in 96-well V-bottomed microtiter plates, centrifuged, and incubated at 37°C for 5 hours. After centrifuging again, 25µl supernatant was harvested and dried onto 96-well solid scintillation microtiter plates before counting the radioactivity in a Topcount Microplate Scintillation Counter (Packard Instrument Company, Meridan, CT).

For determination of antigenic specificity, target cells were infected with vaccinia construct (multiplicity of infection approx. 10:1) and labelled with ⁵¹Cr by a modification of the previously described procedure (17). Both infection and labelling occurred simultaneously for 2.5 hours before washing and using the cells in CTL assays. In some instances target cells were pretreated in V-wells with monoclonal antibodies to HLA-class I (W6/32) or class II (L243) for 30 min at room temperature before adding effector cells.

### Regression assay

The EBV-specific memory CTL response was assessed as previously described (31).

### Limiting dilution cultures

Limiting dilution cultures were established by distributing PBL from the fourth bleed in 24 replicates at 2 x 10⁴ cells per well in 96-well roundbottomed microtitre plates. Doubling dilutions were made to a final concentration of 1.6 x 10² per well. Approximately 1 x 10⁴ γ-irradiated (8000 rads) autologous type B (EW-Ag-876) LCLs were added to each well as both feeder and stimulator cells to a total volume of 100µl. Cultures were fed on days 4 and 7 with 50µl of T cell growth medium and assayed on day 10 or later. The plates were subcultured and maintained in T cell growth medium and stimulated with γ-irradiated EW-Ag-876 LCLs. Initial assays were against type A and type B LCLs and subsequently against peptide-coated PHA-blasts or LCLs. Precursor frequencies were determined by a modification of the method of maximum likelihood estimation (11). Assessment of peptide specific activity. For determination of the antigenic specificity at the peptide level, a set of 20-mer overlapping peptides of EBNA-6B (residues 100-1069 (46)) was prepared using a kit and software distributed by Chiron Mimotopes (Chiron Corporation, Sydney, Australia). Nine-mer and 10-mer overlapping peptides with unblocked C and N termini were manufactured by Chiron Mimotopes. Labelled target cells were preincubated with overlapping peptides in the 96-well trays for 30-60 minutes before adding effectors. Alternatively, labelled targets were preincubated with a high dose of peptide (40-80 µg/ml) then washed twice before using in CTL assays. The washing procedure removes the complication of bystander killing in the latter type of experiments (6).

### Depletion experiments

T-cell cultures were depleted of either CD4 or CD8 cells by pretreating with saturating levels of OKT4 (mouse anti-CD4) or OKT8 (mouse anti-CD8) for 30 min on ice before washing twice and incubating with washed Dynabeads M450 (Sheep anti-mouse IgG coated beads, Dynal, A.S. Oslo. Norway) at 4°C for 60 min with rotation. Beads and adherent cells were removed magnetically and the remaining cells washed once before use in a CTL assay or FACScan analysis.

### RESULTS

### EBV typing of lymphocyte donor

PCR analysis revealed the presence of both type A and type B EBV in blood from donor EW. The presence of both virus types was supported by the observation that both type A and type B spontaneous cell lines grew out although only the former (referred to as EW-spon) was established in long term culture.

### Selection of T-cell cultures for analysis

Regression analysis indicated that the donor had memory CTL activity to both virus types. The number of cultures that could be assessed was therefore minimal compared with the T cell responses of most type A donors investigated by this laboratory. Accordingly, 25 colonies from the third sample were assayed and seven of these selected for further study. Three of these cultures (B4, B5 and B7) were type B specific i.e. they lysed autologous type B cells (> 15% lysis) but not type A (< 10 % lysis); the other four (A3, A9, B6. and B9) were A-B specific i.e. they lysed both type A and type B autologous LCLs (> 15%) (Table 1).

### CTL analysis of vaccinia-infected targets

The seven selected cultures were assayed for CTL activity towards vaccinia-infected autologous type A LCLs (EW-Wil). Four cultures (A3, A9, B4 and B5) gave significant enhancement of CTL activity when vacc-EBNA 6B infected LCLs were compared with control cells or vacc-TK⁻ infected target cells. Furthermore, these cultures reacted poorly with other EBV sequences (EBNA-1. EBNA-2B, EBNA-3B. EBNA-4A. LMP1. LMP2. BZLF1 and BHRF1) expressed by recombinant vaccinia viruses thus indicating specificity for the vacc-EBNA-6B infected target. Cultures B5 (type B specific) and A3 (crossreactive) were selected for more detailed analysis.

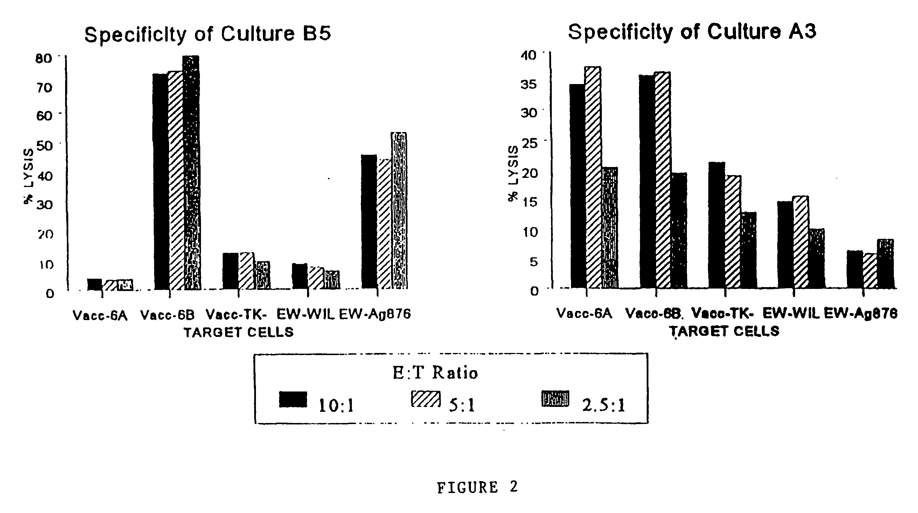

The specificity of cultures A3 and B5 was confirmed in a separate experiment in which EW-Wil target cells were infected with vacc-EBNA-6A and vacc-EBNA-6B. While the CTL activity of colony A3 was enhanced by infection of the target cells with either construct (<10% to 20-37%). the effector function of colony B5 was enhanced only when target cells were infected with vacc-EBNA-6B (< 10% to 73-79.5%) (Fig. 2). These results suggest that culture B5 recognised a type B specific epitope within EBNA-6 while culture A3 recognised a crossreactive epitope within EBNA-6.

### Identification of specific peptide targets of CTL activity

The type B specific culture B5 recognised the peptide corresponding to EBNA-6B residues 210-229 (peptide 12 of the EBNA-6B set) when assayed against individual 20-mer overlapping peptides preincubated with autologous PHA blasts (Fig. 3a). Nine-mer and 10-mer overlapping peptides were then assayed to identify the minimal epitope as the 10-mer QNGALAINTF (residues 213-222: Fig. 3b ). The corresponding peptide from the type A overlapping sequence was not recognised by this culture, thus confirming the type B specificity of these T cells.

Culture A3 was assayed against pools of overlapping 20-mer peptides from EBNA 6B on autologous PHA-blasts in order to identify its peptide target. A pool containing 6 x 20-mer peptides (peptides 19-24 in the EBNA-6B set) from residue 280-349 was recognised by this CTL culture and these peptides were subsequently assayed individually to identify the reactive 20-mer as peptide 19, residues 280-299 (Fig. 4a). In Fig. 4b, the minimal epitope was identified as LLDFVRFMGV corresponding to residues 284 -293 of the EBNA 6A and 6B sequences. This sequence is common to both type A and B of EBV and overlaps with the sequence of the B44-restricted epitope EENLLDFVRF (residues 281 - 290 of EBNA6A and 6B; (7)).

### HLA-restriction of epitopes.

### (a) Type B specific epitope

As Facs assays revealed that cultures recognising the EBNA 6B-encoded sequence QNGALAINTF contained mixtures of CD4⁺, CD4⁺ CD8⁺ and CD8⁺ cells when assayed initially, the possibility that the CTL activity was actually due to a CD4 component needed to be eliminated. Several of these cultures also contained components that killed type A autologous targets, presumably because they were derived from the limiting dilution format and contained a mixture of cells. Depletion and inhibition experiments confirmed that the CD8⁺ cells were the active CTLs.

In the depletion experiments, Dynabeads were used to deplete either CD4⁺ or CD8⁺ cells from reactive cultures. When samples of three different epitope-reactive cultures were tested, depletion of the CD4⁺ cells (which would have included the CD4⁺ CD8⁺ component) did not reduce the CTL activity, but depletion of the CD8⁺ cells completely abrogated the epitope specific activity (Fig. 5). Facs analysis in one of these experiments confirmed that the active fraction contained the CD8⁺ cells and that both the CD4⁺ and the CD4⁺ -CD8⁺ cells had been removed. Fig. 6 illustrates the inhibition of the specific CTL activity of the mixed cultures by anti-class I monoclonal antiserum but not by the anti-class II serum that was positive for DR, DP and DQ alleles. It was concluded therefore that this epitope was Class I-restricted.

In order to identify the restricting allele. LCLs, Burkitt's lymphoma cell lines or PHA blasts of known HLA specificity were incubated with minimalised peptides and washed before using as target cells in CTL assays. As Table 2 indicates, the only target cells killed by the QNGALAINTF specific CTLs were those that were both A201 and B62 (B15) positive. In contrast, targets that were A201 but not B62, and target cells that only shared A24 or B51 alleles with the donor were not killed. It was therefore concluded that the epitope is B62 (B15)-restricted. B62 which comprises at least 5 subtypes has recently been reclassified as part of the B15 family.

### (b) Cross-reactive epitope

In similar experiments, culture A3 consistently recognised cells bearing HLA-A201 but not cells bearing A24, B51, or B62. Furthermore, the CTL activity of this culture was inhibited by anti-class I antibodies but not by anti-class II antibodies. Finally, the A3 minimal epitope corresponds to the motif already identified for HLA-A201 restriction (39). It was therefore concluded that the epitope LLDFVRFMGV corresponding to residues 284-293 in both EBNA-6A and EBNA-6B was HLA Class I A201 restricted.

### Predominance of type B CTL response in limiting dilution cultures and bulk cultures

Limiting dilution culture methodology was used in order to investigate the magnitude of the type B specific CTL response. PBL stimulated by a feeder layer of irradiated autologous type B cells were assayed after 10 days against type A and type B autologous LCLs. The results indicated a higher frequency of T cell precursors killing type B LCLs than of those killing type A LCLs. There were therefore a number of wells at limiting dilutions that were type B specific. In general, the wells manifesting type B specific activity retained this specificity. After minimalising the epitopes as described above, the cultures were retested for epitope specific activity. A frequency of 1/16395 was calculated for the Type B epitope QNGALAINTF compared with 1/304,890 for the A-B specific epitope LLDFVRFMGV when these cultures were assayed 12 weeks later.

Additional EBV CTL epitopes where obtained from other donors using similar methods.

### DISCUSSION

The identification of an EBV type B specific epitope represents the first demonstration of a CTL epitope encoded within type B specific regions of the EBV genome. The epitope was identified as QNGALAINTF corresponding to residues 213-222 of EBNA-6B. The collective data presented here indicates that stimulation of T cells from donor EW by autologous type B infected LCLs reactivated a predominantly type B response rather than a cross-reactive response. The specificity of the type B response could be maintained on extended culture *in vitro.* Clearly the response to QNGALAINTF would seem to be an immunodominant one as indicated by the number of reactive wells in the limiting dilution cultures. It is also interesting to note that a number of type B- specific CTL cultures were established for which specific epitopes could not be identified. It is possible that the target epitopes for these T cells lie within EBNA-4B or outside the regions included in the EBNA-2B, -3B and -6B constructs.

The epitope QNGALAINTF appears to be restricted through HLA B62 (B15), although the subtype specificity has yet to be determined. Motifs for B* 1501 have previously been published (39) and F is commonly found in the last anchor position (9 or 10) as in the epitope identified here. Q or L appear to be the preferred residues at position 2 of B*1501: for the new epitope, Q is at position 1. The corresponding type A sequence for this peptide (QNAARTLNTF) was nonreactive in CTL assays, thus confirming the type B specificity of the epitope.

A second epitope LLDFVRFMGV also represents a previously unrecognised EBV-encoded epitope. This sequence corresponds to residues 284 -293 of both EBNA-6A and 6B and was shown to be restricted by HLA-A201.

An interesting facet of this work was the detection of both type A and type B EBV in the one donor by PCR of both PBL and spontaneous cell lines. Although the available evidence indicates a high prevalence of type B in the community, it is known that in healthy donors type A predominates as the one that is easiest to isolate. Until now, responses to type A virus have therefore been easier to identify.

It was noted in the results section that the donor did not give a particularly active cloning response. It is possible that the type B viruses are less immunogenic than type A. or even that type B EBV has evolved in part to evade the immune system. Type B viruses could conceivably normally be resident in the epithelial compartment rather than the lymphocyte compartment due to their lower transformation efficiency. In this situation, type B epitopes may not be readily presented to the immune system. As already implied, the lower transformation efficiency of type B viruses (42) may hinder reinforcement over time of the immune response to the latency antigens.

Finally, it is important when considering the possibility of vaccinating against EBV to recognise that it may be necessary to vaccinate against both type A and type B. If a patient, particularly a transplant patient at risk of EBV-related post-transplant lymphoma (8), is protected against type A but not type B, there may even be greater risk of clinical disease if subsequently infected with the other virus type. It is particularly important to consider EBV type B epitopes in view of the finding that type B is commonly detected in immunosuppressed individuals. Subunit vaccines against EBV are currently being trialed. In the long term, such vaccines would ideally contain cross-protective specificities. Definition of type B and cross-reactive epitopes allows development of a more effective vaccine, more effective in terms of the range of EBV specificities and HLA types that are covered by the vaccine.

### Bibliography

1. Adldinger. H. K., H. Delius, U. K. Freese, J. Clarke, and G. W. Bornkamm. 1985. A putative transforming gene of Jijoye virus differs from that of Epstein-Barr virus prototypes. Virology 141:221-234.
2. Andrew, M. E., B. E. Coupar, D. B. Boyle, and G. L. Ada. 1987. The roles of influenza virus haemagglutinin and nucleoprotein in protection: analysis using vaccinia virus recombinants. Scand. J. Immunol. 25:21-28.
3. Apolloni. A. and T. B. Sculley. 1994. Detection of A-type and B-type Epstein-Barr virus in throat washings and lymphocytes. Virology 202:978-981.
4. Bogedain. C., H. Wolf, S. Modrow, G. Stuber, and W. Jilg. 1995. Specific cytotoxic T lymphocytes recognize the immediate-early transactivator Zta of Epstein-Barr virus. J. Virol. 69:4872-4879.
5. Buisson, M., P. Morand, O. Genoulaz, M. J. Bourgeat, M. Micoud, and J. M. Seigneurin. 1994. Changes in the dominant Epstein-Barr virus type during human immunodeficiency virus infection. J. Gen. Virol. 75:431-437.
6. Burrows. S. R., A. Fernan, V. Argaet, and A. Suhrbier. 1993. Bystander apoptosis induced by CD8+ cytotoxic T cell (CTL) clones: implications for CTL lytic mechanisms. Int. Immunol. 5:1049-1058.
7. Burrows. S. R., I. S. Misko, T. B. Sculley, C. Schmidt, and D. J. Moss. 1990. An Epstein-Barr virus-specific cytotoxic T-cell epitope present on A- and b-type transformants. J. Virol. 64:3974-3976.
8. Crawford. D. H., J. A. Thomas, G. Janossy, P. Sweny, O. N. Fernando. J. F. Moorhead, and J. H. Thompson. 1980. Epstein Barr virus nuclear antigen positive lymphoma after cyclosporin A treatment in patient with renal allograft. Lancet 1:1355-1356.
9. Dambaugh, T., K. Hennessy, L. Chamnankit, and E. Kieff. 1984. U2 region of Epstein-Barr virus DNA may encode Epstein-Barr nuclear antigen 2. Proc. Natl. Acad. Sci. U. S A. 81:7632-7636.
10. Doherty, P. C., R. A. Tripp, and J. W. Sixbey. 1994. Evasion of host immune responses by tumours and viruses. Ciba. Found. Symp. 187:245-256.
11. Fazekas de St Groth, S. 1982. The evaluation of limiting dilution assays. J. Immunol. Methods 49:R11-23.
12. Gratama, J. W., M. A. Oosterveer. W. Weimar, K. Sintnicolaas, W. Sizoo, R. L. Bolhuis, and I. Ernberg. 1994. Detection of multiple 'Ebnotypes' in individual Epstein-Barr virus carriers following lymphocyte transformation by virus derived from peripheral blood and oropharynx. J. Gen. Virol. 75:85-94.
13. Gregory, C. D., M. Rowe, and A. B. Rickinson. 1990. Different Epstein-Barr virus-B cell interactions in phenotypically distinct clones of a Burkitt's lymphoma cell line. J. Gen. Virol. 71:1481-1495.
14. Henle, G., E. T. Lennette, M. A. Alspaugh, and W. Henle. 1979. Rheumatoid factor as a cause of positive reactions in tests for epstein-barr virus-specific IgM antibodies. Clin. Exp. Immunol. 36:415-422.
15. Henle, W. and G. Henle. 1979. Seroepidemiology of the Virus, p. 61-78. In M. A. Epstein and B. G. Achong (eds.), The Epstein-Barr Virus. Springer-Verlag, Berlin.
16. Hu, L. F.. J. Minarovits, S. L. Cao. B. Contreras-Salazar. L. Rymo, K. Falk, G. Klein, and I. Ernberg. 1991. Variable expression of latent membrane protein in nasopharyngeal carcinoma can be related to methylation status of the Epstein-Barr virus BNLF-1 5'-flanking region. J. Virol. 65:1558-1567.
17. Khanna, R., S. R. Burrows, M. G. Kurilla, C. A. Jacob, I. S. Misko, T. B. Sculley, E. Kieff, and D. J. Moss. 1992. Localization of Epstein-Barr virus cytotoxic T-cell epitopes using recombinant vaccinia: implications for vaccine development. J. Exp. Med. 176:169-176.
18. Khanna, R., S. R. Burrows, and D. J. Moss. 1995. Immune regulation in Epstein-Barr virus-associated diseases. Microbiol. Rev. 59:387-405.
19. Khanna, R., C. A. Jacob, S. R. Burrows, M. G. Kurilla, E. Kieff, I. S. Misko, and D. J. Moss. 1991. Expression of Epstein-Barr virus nuclear antigens in anti-IgM-stimulated B cells following recombinant vaccinia infection and their recognition by human cytotoxic T-cells. Immunology 74:504-510.
20. Kyaw, M. T., L. Hurren, L. Evans, D. J. Moss, D. A. Cooper. E. Benson, D. Esmore, and T. B. Sculley. 1992. Expression of B-type Epstein-Barr virus in HIV-infected patients and cardiac transplant recipients. AIDS Res. Hum. Retroviruses 8:1869-1874.
21. Landau, Z., R. Gross, A. Sanilevich. A. Friedmann, and S. Mitrani Rosenbaum. 1994. Presence of infective Epstein-Barr virus in the urine of patients with infectious mononucleosis. J. Med. Virol. 44:229-233.
22. Laroche. C., E. B. Drouet. P. Brousset. C. Pain. A. Boibieux, F. Biron, J. Icart, G. A. Denoyel, and A. Niveleau. 1995. Measurement by the polymerase chain reaction of the Epstein-Barr virus load in infectious mononucleosis and AIDS-related non-Hodgkin's lymphomas. J. Med. Virol. 46:66-74.
23. Lear, A. L., M. Rowe, M. G. Kurilla, S. Lee, S. Henderson. E. Kieff, and A. B. Rickinson. 1992. The Epstein-Barr virus (EBV) nuclear antigen 1 BamHI F promoter is activated on entry of EBV-transformed B cells into the lytic cycle. J. Virol. 66:7461-7468.
24. Lewin, N.. P. Aman, M. G. Masucci, E. Klein, G. Klein, B. Oberg, H. Strander, W. Henle, and G. Henle. 1987. Characterization of EBV-carrying B-cell populations in healthy seropositive individuals with regard to density, release of transforming virus and spontaneous outgrowth. Int. J. Cancer 39:472-476.
25. Lung, M. L., R. S. Chang, M. L. Huang, H. Y. Guo, D. Choy, J. Sham, S. Y. Tsao, P. Cheng, and M. H. Ng. 1990. Epstein-Barr virus genotypes associated with nasopharyngeal carcinoma in southern China. Virology 177:44-53.
26. Miller, C. L., J. H. Lee, E. Kieff, and R. Longnecker. 1994. An integral membrane protein (lmp2) blocks reactivation of epstein-barr virus from latency following surface immunoglobulin crosslinking. Proc. Natl. Acad. Sci. U. S A. 91:772-776.
27. Miller, N. and L. M. Hutt-Fletcher. 1988. A monoclonal antibody to glycoprotein gp85 inhibits fusion but not attachment of Epstein-Barr virus. J. Virol. 62:2366-2372.
28. Misko, I. S., T. D. Soszynski, R. G. Kane, and J. H. Pope. 1984. Factors influencing the human cytotoxic T-cell response to autologous lymphoblastoid cell lines in vitro. Clin. Immunol. Immunopathol. 32:285-297.
29. Miyashita, E. M., B. Yang, K. M. Lam, D. H. Crawford, and D. A. Thorley-Lawson. 1995. A novel form of Epstein-Barr virus latency in normal B cells in vivo. Cell 80:593-601.
30. Moss, D. J., I. S. Misko, S. R. Burrows, K. Burman, R. McCarthy, and T. B. Sculley. 1988. Cytotoxic T-cell clones discriminate between A- and B-type Epstein-Barr virus transformants. Nature 331:719-721.
31. Moss, D. J., A. B. Rickinson, and J. H. Pope. 1978. Long-term T-cell-mediated immunity to Epstein-Barr virus in man. I. Complete regression of virus-induced transformation in cultures of seropositive donor leukocytes. Int. J. Cancer 22:662-668.
32. Murray. R. J., M. G. Kurilla, J. M. Brooks, W. A. Thomas, M. Rowe, E. Kieff, and A. B. Rickinson. 1992. Identification of target antigens for the human cytotoxic T-cell response to Epstein-Barr virus (EBV): implications for the immune control of EBV-positive malignancies. J. Exp. Med. 176:157-168.
33. Murray, R. J., M. G. Kurilla, H. M. Griffin, J. M. Brooks, M. Mackett, J. R. Arrand, M. Rowe, S. R. Burrows, D. J. Moss, E. Kieff, and A. B. Rickinson. 1990. Human cytotoxic T-cell responses against Epstein-Barr virus nuclear antigens demonstrated by using recombinant vaccinia viruses. Proc. Natl. Acad. Sci. U. S A. 87:2906-2910.
34. Nilsson, K., G. Klein, W. Henle, and G. Henle. 1971. The establishment of lymphoblastoid lines from adult and fetal human lymphoid tissue and its dependence on EBV. Int. J. Cancer 8:443-450.
35. Okano, M., S. Matsumoto, T. Osato, Y. Sakiyama, G. M. Thiele, and D. T. Purtilo. 1991. Severe chronic active Epstein-Barr virus infection syndrome. Clin. Microbiol. Rev. 4:129-135.
36. Polack, A., G. Hartl, U. Zimber, U. K. Freese, G. Laux, K. Takaki, B. Hohn, L. Gissmann, and G. W. Bornkamm. 1984. A complete set of overlapping cosmid clones of M-ABA virus derived from nasopharyngeal carcinoma and its similarity to other Epstein-Barr virus isolates. Gene 27:279-288.
37. Pope, J. H., B. G. Achong, M. A. Epstein, and J. Biddulph. 1967. Burkitt lymphoma in New Guinea: establishment of a line of lymphoblasts in vitro and description of their fine structure. J. Natl. Cancer Inst. 39:933-945.
38. Pope, J. H., M. K. Horne, and W. Scott. 1968. Transformation of fetal human leucocytes in vitro by filtrates of a human leukemic cell line containing herpes-like virus. Int. J. Cancer 3:857-844.
39. Rammensee, H. G.. T. Friede, and S. Stevanoviic. 1995. MHC ligands and peptide motifs: first listing. Immunogenetics 41:178-228.
40. Ressing, M. E., A. Sette, R. M. Brandt, J. Ruppert, P. A. Wentworth, M. Hartman, C. Oseroff, H. M. Grey, C. J. Melief, and W. M. Kast. 1995. Human CTL epitopes encoded by human papillomavirus type 16 E6 and E7 identified through in vivo and in vitro immunogenicity studies of HLA-A*0201-binding peptides. J. Immunol. 154:5934-5943.
41. Rickinson, A. B., M. Rowe, I. J. Hart, Q. Y. Yao, L. E. Henderson, H. Rabin, and M. A. Epstein. 1984. T-cell-mediated regression of spontaneous and of Epstein-Barr virus-induced B-cell transformation in vitro: studies with cyclosporin A. Cell. Immunol. 87:646-658.
42. Rickinson, A. B., L. S. Young, and M. Rowe. 1987. Influence of the Epstein-Barr virus nuclear antigen EBNA 2 on the growth phenotype of virus-transformed B cells. J. Virol. 61:1310-1317.
43. Rooney, C. M., A. B. Rickinson, D. J. Moss, G. M. Lenoir, and M. A. Epstein. 1984. Paired Epstein-Barr virus-carrying lymphoma and lymphoblastoid cell lines from Burkitt's lymphoma patients: comparative sensitivity to non-specific and to allo-specific cytotoxic responses in vitro. Int. J. Cancer 34:339-348.
44. Rooney, C. M., M. Rowe, L. E. Wallace, and A. B. Rickinson. 1985. Epstein-Barr virus-positive Burkitt's lymphoma cells not recognized by virus-specific T-cell surveillance. Nature 317:629-631.
45. Rosenberg, S. A., E. A. Grimm, M. McGrogan, M. Doyle, E. Kawasaki, K. Koths, and D. F. Mark. 1984. Biological activity of recombinant human interleukin-2 produced in Escherichia coli. Science 223:1412-1414.
46. Sample. J. and E. Kieff. 1990. Transcription of the Epstein-Barr virus genome during latency in growth-transformed lymphocytes. J. Virol. 64:1667-1674.
47. Sculley, T. B., A. Apolloni, L. Hurren, D. J. Moss, and D. A. Cooper. 1990. Coinfection with A- and B-type Epstein-Barr virus in human immunodeficiency virus-positive subjects. J. Infect. Dis. 162:643-648.
48. Sculley, T. B., A. Apolloni, R. Stumm, D. J. Moss, N. Mueller-Lantzsch, I. S. Misko, and D. A. Cooper. 1989. Expression of Epstein-Barr virus nuclear antigens 3, 4, and 6 are altered in cell lines containing B-type virus. Virology 171:401-408.
49. Selin, L. K., S. R. Nahill, and R. M. Welsh. 1994. Cross-reactivities in memory cytotoxic T lymphocyte recognition of heterologous viruses. J. Exp. Med. 179:1933-1943.
50. Silins, S. L., V. P. Argaet, and T. B. Sculley. 1992. Isolation of Epstein-Barr virus genomes using pulse-field gel electrophoresis. Nucleic Acids Res. 20:2901
51. Sixbey, J. W., P. Shirley, P. J. Chesney, D. M. Buntin, and L. Resnick. 1989. Detection of a second widespread strain of Epstein-Barr virus. Lancet 2:761-765.
52. Sumaya, C. V. and Y. Ench. 1985. Epstein-Barr virus infectious mononucleosis in children. I. Clinical and general laboratory findings. Pediatrics 75:1003-1010.
53. Tomkinson, B. E., D. K. Wagner, D. L. Nelson, and J. L. Sullivan. 1987. Activated lymphocytes during acute Epstein-Barr virus infection. J. Immunol. 139:3802-3807.
54. Tussey, L. G., S. Rowland Jones, T. S. Zheng, M. J. Androlewicz, P. Cresswell, J. A. Frelinger, and A. J. McMichael. 1995. Different MHC class I alleles compete for presentation of overlapping viral epitopes. Immunity. 3:65-77.
55. Wagner, H. J., G. Bein, A. Bitsch, and H. Kirchner. 1992. Detection and quantification of latently infected B lymphocytes in Epstein-Barr virus-seropositive, healthy individuals by polymerase chain reaction. J. Clin. Microbiol. 30:2826-2829.
56. Walling, D. M., S. N. Edmiston, J. W. Sixbey, M. Abdel-Hamid, L. Resnick, and N. Raab-Traub. 1992. Coinfection with multiple strains of the Epstein-Barr virus in human immunodeficiency virus-associated hairy leukoplakia. Proc. Natl. Acad. Sci. U. S A. 89:6560-6564.
57. Wang, A., S. D. Lu, and D. F. Mark. 1984. Site-specific mutagenesis of the human interleukin-2 gene: structure-function analysis of the cysteine residues. Science 224:1431-1433.
58. Yamamoto, M., H. Kimura, T. Hironaka, K. Hirai, S. Hasegawa, K. Kuzushima, M. Shibata, and T. Morishima. 1995. Detection and quantification of virus DNA in plasma of patients with Epstein-Barr virus-associated diseases. J. Clin. Microbiol. 33:1765-1768.
59. Yao, Q. Y., A. B. Rickinson, and M. A. Epstein. 1985. A re-examination of the Epstein-Barr virus carrier state in healthy seropositive individuals. Int. J. Cancer 35:35-42.

**Table 1. Initial Selection of CTL Cultures**

| Culture No. # | Target Cells (% Lysis) | | | Facs Markers (% +ve) | | | | Specificity * |
|---|---|---|---|---|---|---|---|---|
| | EW-Wil (type A) | EW-Ag-876 (type B) | K562 (NK sensitive) | CD3⁺ | CD4⁺ CD3⁺ | CD8⁺ CD3⁺ | CD4⁺CD8⁺ CD3⁺ | |
| A3 | 33.8 | 17.0 | 8.2 | 97.5 | 0.3 | 85 | 2 | A-B |
| A9 | 17.7 | 10.0 | 6.1 | 89 | 2 | 82 | 10 | A-B |
| B4 | 6.6 | 16.6 | 4.3 | 93 | 2 | 70 | 0 | B |
| B5 | 5.9 | 23.8 | 7.8 | 95 | 4 | 90.5 | 5.1 | B |
| B6 | 18.5 | 31.3 | 9.6 | 80 | 2 | 66 | 7 | A-B |
| B7 | 8.1 | 30.2 | 4.1 | 86 | 80 | 0 | 1 | B |
| B9 | 16.8 | 7.7 | 4.1 | 87 | 0 | 96 | 0 | A-B |
| # Cultures designated A were generated by stimulation with type A autologous LCLs; cultures designated B were derived by stimulation with type B autologous LCLs | | | | | | | | |
| * A-B specificity: > 10% of both type A and B autologous cell lines; type B specificity: < 10% lysis of type A and > 15% lysis of type B autologous cell lines | | | | | | | | |

**Table 2. HLA-Restriction of type B-specific and crossreactive epitopes**

| TARGET CELLS (Transforming isolate) | EBV TYPE | CLASS I ALLELES | | | | B-type epitope CTL | | Cross-reactive epitope CTL | |
|---|---|---|---|---|---|---|---|---|---|
| | | | | | | -Pep | +Pep | -Pep² | +Pep |
| EW-PHA BLASTS | - | A2 | A24 | B51 | B62 | - | + | - | + + |
| RM-LCL (WIL) | A | A101 | A2 | B8 | B62 | - | + | - | ++ |
| LP-LCL (WIL) | A | A2 | A32 | B35 | B62 | - | + | +,- | + + |
| PGP-LCL(PUY) | A | A1 | A24 | B8 | B14 | - | - | - | - |
| WW II-LCL (WWII) | A | A11 | A24 | B18 | B39 | - | - | - | ((+)) |
| IM-LCL (GOR) | B | A1 | A11 | B51 | B8 | - | NT | - | NT |
| AS-LCL | A,B | A201 | A24 | B51 | B62 | - | NT | NT | NT |
| SB-PHA | - | A201 | A201 | B35 | B57 | - | - | - | ++ |
| CHEP (BL) ¹ | A | A2 | A3 | B7 | | - | - | - | - |
| MUTU (BL) | A | A1 | A2 | B45 | | - | - | - | + |
| TM-LCL (Wil) | A | A11 | A32 | B35 | B15 | - | - | - | - |
| JAP-LCL (Wil) | A | A2 | A28 | B8 | B15 | - | + | - | + |

| | | | | | | | | | |
|---|---|---|---|---|---|---|---|---|---|
| 1: BL = Burkitt's Lymphoma cell lines - used as targets in peptide experiments as the level of background killing is low 2: Clone A3 was originally selected on the basis of CTL activity towards both EW-Wil (type A) and EW-Ag-876 (type B) LCL's, but in the above experiments this clone had low activity only towards heterologous Wil-transformed LCL's. Cultures that recognised either the crossreactive epitope or the type B-specific epitope were tested in CTL assays against heterologous cell lines with or without specific peptide | | | | | | | | | |

**Table 3**

| EBV Antigen | Peptide Epitope | HLA Restriction | EBV Type |
|---|---|---|---|
| EBNA3A | QVKWRMTTL | HLA B8 | Type 2 |
| EBNA3A | VFSDGRVAC | HLA A29 | Type 1 |
| EBNA3A | VPAPAGPIV | HLA B7 | Type 1 |
| EBNA3B | TYSAGIVQI | HLA A24 | Type 1 |
| EBNA3C | LLDFVRFMGV | HLA A2 | Type 1 & 2 |
| EBNA3C | QNGALAINTF | HLA B62 | Type 2 |
| EBNA3A | VSSDGRVAC | HLA A29 | Type 1 |
| EBNA3A | VSSEGRVAC | HLA A29 | Type 1 |
| EBNA3A | VSSDGRVPC | HLA A29 | Type 1 |
| EBNA3A | VSSDGLVAC | HLA A29 | Type 1 |
| EBNA3A | VSSDGQVAC | HLA A29 | Type 1 |
| EBNA3A | VSSDGRWC | HLA A29 | Type 1 |
| EBNA3A | VPAPPVGPIV | HLA B7 | Type 1 |
| EBNA3A | VEITPYEPTG | HLA B44 | Type 1 |
| EBNA3A | VEITPYEPTW | HLA B44 | Type 1 |
| EBNA3A | VELTPYKPTW | HLA B44 | Type 1 |
| EBNA3C | RRIYDLIKL | HLA B27 | Type 1 |
| EBNA3C | RKIYDLIEL | HLA B27 | Type 1 |
| LMP2A | PYLFWLAGI | HLA A23 | |

**Table 4**

| EBV Antigen | Peptide Epitope | HLA Restriction | EBV Type | Reference |
|---|---|---|---|---|
| EBNA1 | TSLYNLRRGTALA | HLA DR1 | Type 1 & 2 | (Khanna 1995A) |
| EBNA2 | DTPLIPLTIF | HLA B51/A2 | Type 1 | (Schmidt 1991) |
| EBNA2 | TVFYNIPPNIPL | HLA DQ2 | Type 1 | WO 95/24925 |
| EBNA3A | VEITPYKPTW | HLA B44 | Type 1 | WO 95/24925 |
| EBNA4 | VSFIEFVGW | HLA B57 | Type 1 | WO 95/24925 |
| EBNA6 | FRKAQIQGL | HLA B57 | Type 1 | WO 95/24925 |
| EBNA3A | FLRGRAYGL | HLA B8 | Type 1 | (Burrows 1992) |
| EBNA3A | QAKWRLQTL | HLA B8 | Type 1 | (Burrows 1994B) |
| EBNA3A | SVRDRLARL | HLA A2 | Type 1 & 2 | (Burrows 1994B) |
| EBNA3A | YPLHEQHGM | HLA B35 | Type 1 | (Burrows 1994B) |
| EBNA3A | HLAAQGMAY | HLA? | Type 1 | (Burrows 1994B) |
| EBNA3A | RPPIFIRRL | HLA B7 | Type 1 | (Hill 1995) |
| EBNA3A | RLRAEAGVK | HLA A3 | Type 1 | (Hill 1995) |
| EBNA3B | IVTDFSVIK | HLA A11 | Type 1 | (Gavioli 1993A) |
| EBNA3B | AVFDRKSDAK | HLA A11 | Type 1 | (Gavioli 1993A) |
| EBNA3A | *NPTQAPVIQLVHAVY | HLA A11 | Type 1 | (Gavioli 1993A) |
| EBNA3A | *LPGPQVTAVLLHEES | HLA A11 | Type 1 | (Gavioli 1993A) |
| EBNA3A | *DEPASTEPVHDQLL | HLA A11 | Type 1 | (Gavioli 1993A) |
| EBNA3A | RYSIFFDY | HLA 24 | Type 1 | (Burrows 1992) |
| EBNA3B | AVLLHEESM | HLA B35 | Type 1 | (Khanna 1995) |
| EBNA3B | RRARSLSAERY | HLA B27 | Type 1 | (Hill 1995) |
| EBNA3C | EENLLDFVRF | HLA B44 | Type 1 & 2 | (Burrows 1990) |
| EBNA3C | KEHVIQNAF | HLA B44 | Type 1 | (Khanna 1992) |
| EBNA3C | RRIYDLIEL | HLA B27 | Type 1 | (Brooks 1993B) |
| EBNA3C | QPRAPIRPI | HLA B7 | Type 1 & 2 | (Hill 1995) |
| EBNA3C | EGGVGWRHW | B44 | | (Morgan 1996) |
| LMP2A | CLGGLLTMV | HLA A2 | Type 1 & 2 | (Lee 1993B) |
| LMP2A | RRRWRRLTV | HLA B27 | Type 1 | (Brooks 1993B) |
| Zta | RAKFKQLL | HLA B8 | | (Bogedain 1995) |
| Zta | *RKCCRAKFKQLLQHYR | HLA Cw6 | | (Bogedain 1995) |

### References and legend for Table 4

* Epitope sequence not minimalised
Bogedain, C.. Wolf, H., Modrow, S., Stuber, G., and Jilg, W. (1995).Specific cytotoxic T lymphocytes recognize the immediate-early transactivator Zta of Epstein-Barr virus. J. Virol. 69, 4872-4879.
Brooks, J.M., Murray, R.J., Thomas, W.A., Kurilla, M.G., and Rickinson. A.B. (1993). Different HLA-B27 subtypes present the same immunodominant Epstein-Barr virus peptide. J. Exp. Med. 178, 879-887.
Burrows, S.R., Misko, I.S., Sculley, T.B.. Schmidt, C., and Moss, D.J. (1990). An Epstein-Barr virus-specific cytotoxic T-cell epitope present on A- and b-type transformants. J. Virol. 64, 3974-3976.
Burrows, S.R., Rodda, S.J., Suhrbier, A., Geysen, H.M., and Moss, D.J. (1992). The specificity of recognition of a cytotoxic T lymphocyte epitope. Eur. J. Immunol. 22, 191-195.
Burrows, S.R., Gardner, J., Khanna, R., Steward, T., Moss, D.J.. Rodda, S., and Suhrbier. A. (1994). Five new cytotoxic T cell epitopes identified within Epstein-Barr virus nuclear antigen 3. J. Gen. Virol. 75, 2489-2493.
Gavioli, R., Kurilla, M.G.. de Campos-Lima, P.O.. Wallace, L.E., Dolcetti. R., Murray, R.J.. Rickinson, A.B., and Masucci. M.G. (1993). Multiple HLA All-restricted cytotoxic T-lymphocyte epitopes of different immunogenicities in the Epstein-Barr virus-encoded nuclear antigen 4. J. Virol. 67, 1572-1578.
Hill, A., Worth, A., Elliott, T., Rowland-Jones, S., Brooks, J., Rickinson. A., and McMichael. A. (1995). Characterization of two Epstein-Barr virus epitopes restricted by HLA-B7. Eur. J. Immunol. 25, 18-24.
Hill, A.B., Lee, S.P., Haurum, J.S., Murray, N., Yao, Q.Y., Rowe, M., Signoret, N., Rickinson. A.B., and McMichael, A.J. (1995). Class I major histocompatibility complex-restricted cytotoxic T lymphocytes specific for Epstein-Barr virus (EBV)-transformed B lymphoblastoid cell lines against which they were raised. J. Exp. Med. 181, 2221-2228.
Khanna, R., Burrows, S.R., Kurilla, M.G., Jacob, C.A., Misko, I.S., Sculley, T.B., Kieff, E., and Moss, D.J. (1992). Localization of Epstein-Barr virus cytotoxic T-cell epitopes using recombinant vaccinia: implications for vaccine development. J. Exp. Med. 176, 169-176.
Khanna. R., Burrows, S.R., and Moss, D.J. (1995a). Immune regulation in Epstein-Barr virus-associated diseases. Microbiol. Rev. 59, 387-405.
Khanna, R., Burrows, S.R., Steigerwald Mullen, P.M., Thomson, S.A., Kurilla. M.G., and Moss. D.J. (1995b). Isolation of cytotoxic T lymphocytes from healthy seropositive individuals specific for peptide epitopes from Epstein-Barr virus nuclear antigen 1: implications for viral persistence and tumor surveillance. Virology 214, 633-637.
Lee, S.P., Thomas, W.A., Murray, R.J., Khanim, F., Kaur, S., Young, L.S., Rowe, M., Kurilla, M., and Rickinson, A.B. (1993). HLA A2.1-restricted cytotoxic T-cells recognizing a range of Epstein-Barr virus isolates through a defined epitope in latent membrane. J. Virol. 67, 7428-7435.
Morgan. S.M., Wilkinson, G.W., Floettmann, E., Blake. N., and Rickinson, A.B. (1996). A recombinant adenovirus expressing an Epstein-Barr virus (EBV) target antigen can selectively reactivate rare components of EBV cytotoxic T-lymphocyte memory in vitro. J Virol 70, 2394-2402.,
Schmidt. C., Burrows, S.R., Sculley, T.B., Moss, D.J., and Misko, I.S. (1991). Nonresponsiveness to an immunodominant Epstein-Barr virus-encoded cytotoxic T-lymphocyte epitope in nuclear antigen 3a: implications for vaccine strategies. Proc. Natl. Acad. Sci. U. S A. 88, 9478-9482.

## Claims

1. A cytotoxic Epstein-Barr virus T-cell epitope, the epitope being selected from the group consisting of QVKWRMTTL and QNGALAINTF.

2. An EBV type B-specific subunit vaccine, the vaccine including at least one T-cell epitope selected from the group consisting of QVKWRMTTL and QNGALAINTF.

3. An EBV type B-specifc vaccine including the cytotoxic T-cell epitopes QVKWRMTTL and QNGALAINTF.

4. A vaccine as claimed in claims 2 or 3 in which the vaccine further includes at least one antigen in addition to the cytotoxic T-cell epitope.

5. A vaccine as claimed in claim 4 in which the antigen is selected from the group consisting of tetanus toxoid, diphtheria toxoid, Bordetella pertussis antigens, poliovirus antigens, gp350 protein, and combinations thereof.

6. A vaccine as claimed in claim 5 in which the antigen is tetanus toxoid.

7. A vaccine as claimed in any one of claims 2 to 6 in which the vaccine comprises a water-in-oil formulation.

8. An EBV type B-specific nucleic acid vaccine, the vaccine including a nucleic acid sequence encoding cytotoxic T-cell epitopes selected from the group consisting of QVKWRMTRL and QNGALAINTF.

9. An EBV type B-specific nucleic acid vaccine, the vaccine including a nucleic acid sequence encoding the cytotoxic T-cell epitopes QVKWRMTTL and QNGALAINTF.

10. A cytotoxic Epstein-Barr virus T-cell epitope as defined in claim 1 for use in medicine.

11. The use of a cytotoxic Epstein-Barr virus T-cell epitope as defined in claim 1 in the preparation of an EBV type B-specific vaccine for the prophylaxis or therapy of an infection caused by Epstein-Barr virus type B.

12. The use of an isolated nucleic acid encoding a cytotoxic Epstein-Barr virus T-cell epitope selected from the group consisting of QVKWRMTTL and QNGALAINTF in the preparation of an EBV type B-specific nucleic acid vaccine for the prophylaxis or therapy of an infection caused by Epstein-Barr virus type B.

13. A vaccine as claimed in claims 2 or 3 further comprising a helper epitope.

14. The use of a cytotoxic Epstein-Barr virus T-cell epitope as defined in claim 1 in combination with an antigen in the preparation of an EBV type B-specific vaccine for the prophylaxis or therapy of an infection caused by Epstein-Barr virus type B.

15. The use of a cytotoxic Epstein-Barr virus T-cell epitope as defined in claim 1 in combination with a helper epitope of an EBV type B-specific in the preparation of a vaccine for the prophylaxis or therapy of an infection caused by Epstein-Barr virus type B.

## Patentansprüche

1. Zytotoxisches T-Zellen-Epitop des Epstein-Barr-Virus, wobei das Epitop aus der Gruppe bestehend aus QVKWRMTTL und QNGALAINTF ausgewählt ist.

2. EBV-Typ-B-spezifisches Untereinheit-Vakzin, wobei das Vakzin zumindest ein T-Zellen-Epitop ausgewählt aus der Gruppe bestehend aus QVKWRMTTL und QNGALAINTF umfasst.

3. EBV-Typ-B-spezifisches Vakzin, das die zytotoxischen T-Zellen-Epitope QVKWRMTTL und QNGALAINTF umfasst.

4. Vakzin nach Anspruch 2 oder 3, wobei das Vakzin weiters zumindest ein Antigen zusätzlich zum zytotoxischen T-Zellen-Epitop umfasst.

5. Vakzin nach Anspruch 4, wobei das Antigen aus der Gruppe bestehend aus Tetanustoxoid, Diphtherietoxoid, Bordetellapertussis-Antigene, Poliovirus-Antigene, gp350-Protein und Kombinatio-nen davon ausgewählt ist.

6. Vakzin nach Anspruch 5, wobei das Antigen Tetanustoxoid ist.

7. Vakzin nach einem der Ansprüche 2 bis 6, wobei das Vakzin eine Wasser-in-Öl-Formulierung umfasst.

8. EBV-Typ-B-spezifisches Nukleinsäurevakzin, wobei das Vakzin eine für zytotoxische T-Zellen-Epitope kodierende Nukleinsäuresequenz umfasst, die aus der Gruppe bestehend aus QVKMRMTTL und QNGALAINTF ausgewählt ist.

9. EBV-Typ-B-spezifisches Nukleinsäurevakzin, wobei das Vakzin eine Nukleinsäuresequenz aufweist, die für die zytotoxischen T-Zellen-Epitope QVKWRMTTL und QNGALAINTF kodiert.

10. Zytotoxisches T-Zellen-Epitop des Epstein-Barr-Virus nach Anspruch 1 zur Verwendung in der Medizin.

11. Verwendung eines zytotoxischen T-Zellen-Epitops des Epstein-Barr-Virus nach Anspruch 1 bei der Herstellung eines EBV-Typ-B-spezifischen Vakzins zur Vorbeugung oder Therapie einer durch den Epstein-Barr-Virus Typ B hervorgerufenen Infektion.

12. Verwendung einer isolierten Nukleinsäure, die für ein zytotoxisches T-Zellen-Epitop des Epstein-Barr-Virus kodiert, ausgewählt aus der Gruppe bestehend aus QVKWRMTTL und QNGALAINTF, bei der Herstellung eines EBV-Typ-B-spezifischen Nukleinsäurevakzins zur Vorbeugung oder Therapie einer durch den Epstein-Barr-Virus Typ B hervorgerufenen Infektion.

13. Vakzin nach Anspruch 2 oder 3, das weiters ein Helferepitop umfasst.

14. Verwendung eines zytotoxischen T-Zellen-Epitops des Epstein-Barr-Virus nach Anspruch 1 in Kombination mit einem Antigen bei der Herstellung eines EBV-Typ-B-spezifischen Vakzins zur Vorbeugung oder Therapie einer durch den Epstein-Barr-Virus Typ B hervorgerufenen Infektion.

15. Verwendung eines zytotoxischen T-Zellen-Epitops des Epstein-Barr-Virus nach Anspruch 1 in Kombination mit einem Helferepitop eines EBV-Typ-B-spezifischen (Objekt fehlt!!) bei der Herstellung eines Vakzins zur Vorbeugung oder Therapie einer durch den Epstein-Barr-Virus Typ B hervorgerufenen Infektion.

## Revendications

1. Epitope de lymphocyte T cytotoxique de virus d'Epstein-Barr, ledit épitope étant sélectionné parmi le groupe constitué de QVKWRMTTL et de QNGALAINTF.

2. Vaccin de sous-unité spécifique d'EBV de type B, ledit vaccin incluant au moins un épitope de lymphocyte T sélectionné parmi le groupe constitué de QVKWRMTTL et de QNGALAINTF.

3. Vaccin spécifique d'EBV de type B, incluant les épitopes de lymphocytes T cytotoxiques QVKWRMTTL et QNGALAINTF.

4. Vaccin selon les revendications 2 ou 3, dans lequel le vaccin inclut en outre au moins un antigène en complément de l'épitope de lymphocyte T cytotoxique.

5. Vaccin selon la revendication 4, dans lequel l'antigène est sélectionné parmi le groupe constitué de l'anatoxine tétanique, l'anatoxine diphtérique, les antigènes de Bordetella pertussis, les antigènes de poliovirus, la protéine gp350 et les associations de ces composés.

6. Vaccin selon la revendication 5, dans lequel l'antigène est l'anatoxine tétanique.

7. Vaccin selon l'une quelconque des revendications 2 à 6, dans lequel le vaccin comprend une formulation eau-dans-l'huile.

8. Vaccin d'acide nucléique spécifique d'EBV de type B, ledit vaccin incluant une séquence d'acide nucléique codant pour des épitopes de lymphocytes T cytotoxiques sélectionnés parmi le groupe constitué de QVKWRMTTL et de QNGALAINTF.

9. Vaccin d'acide nucléique spécifique d'EBV de type B, ledit vaccin incluant une séquence d'acide nucléique codant pour les épitopes de lymphocytes T cytotoxiques QVKWRMTTL et QNGALAINTF.

10. Epitope de lymphocyte T cytotoxique de virus d'Epstein-Barr selon la revendication 1, pour usage en médecine.

11. Utilisation d'un épitope de lymphocyte T cytotoxique de virus d'Epstein-Barr selon la revendication 1 dans la préparation d'un vaccin spécifique de l'EBV de type B pour la prophylaxie ou le traitement d'une infection causée par le virus d'Epstein-Barr de type B.

12. Utilisation d'un acide nucléique isolé codant pour un épitope de lymphocyte T cytotoxique de virus d'Epstein-Barr sélectionné parmi le groupe constitué de QVKWRMTTL et QNGALAINTF, dans la préparation d'un vaccin d'acide nucléique spécifique de l'EBV de type B pour la prophylaxie ou le traitement d'une infection causée par le virus d'Epstein-Barr de type B.

13. Vaccin selon les revendications 2 ou 3, comprenant en outre un épitope auxiliaire *(helper).*

14. Utilisation d'un épitope de lymphocyte T cytotoxique de virus d'Epstein-Barr selon la revendication 1 en association avec un antigène dans la préparation d'un vaccin spécifique de l'EBV de type B pour la prophylaxie ou le traitement d'une infection causée par le virus d'Epstein-Barr de type B.

15. Utilisation d'un épitope de lymphocyte T cytotoxique de virus d'Epstein-Barr selon la revendication 1 en association avec un épitope auxiliaire spécifique d'un EBV de type B dans la préparation d'un vaccin pour la prophylaxie ou le traitement d'une infection causée par le virus d'Epstein-Barr de type B.
